(19) Europäisches Patentamt
European Patent Office
Office européen des brevets

(11) EP 1 388 338 B1

(12) **EUROPÄISCHE PATENTSCHRIFT**

(45) Veröffentlichungstag und Bekanntmachung des Hinweises auf die Patenterteilung:
**29.09.2010 Patentblatt 2010/39**

(51) Int Cl.:
***A61K 8/04*** *(2006.01)* ***A61K 8/49*** *(2006.01)*
***A61K 8/60*** *(2006.01)* ***A61Q 17/04*** *(2006.01)*

(21) Anmeldenummer: **03014581.7**

(22) Anmeldetag: **07.07.2003**

(54) **Schäumbare Sonnenschutzzubereitungen**

Foamable sunscreen compositions

Compositions anti-solaires moussables

(84) Benannte Vertragsstaaten:
**AT BE BG CH CY CZ DE DK EE ES FI FR GB GR HU IE IT LI LU MC NL PT RO SE SI SK TR**

(30) Priorität: **07.08.2002 DE 10236064**

(43) Veröffentlichungstag der Anmeldung:
**11.02.2004 Patentblatt 2004/07**

(73) Patentinhaber: **Beiersdorf AG**
**20245 Hamburg (DE)**

(72) Erfinder:
- **Nielsen, Jens**
  **24558 Henstedt-Ulzburg (DE)**
- **Grotelüschen, Birgit**
  **22459 Hamburg (DE)**
- **Menzel, Norbert**
  **21244 Buchholz (DE)**
- **Schulz, Jens Dr.**
  **22869 Schenefeld (DE)**
- **Hahl, Michael**
  **20253 Hamburg (DE)**

(56) Entgegenhaltungen:

| | |
|---|---|
| **EP-A- 0 821 945** | **EP-A- 1 093 796** |
| **EP-A- 1 277 460** | **EP-A- 1 319 395** |
| **EP-A1- 0 579 455** | **WO-A-97/03643** |
| **WO-A-03/011238** | **WO-A-03/026595** |
| **WO-A2-01/64164** | **DE-A- 19 723 733** |
| **DE-A- 19 726 785** | **DE-A- 19 945 577** |
| **DE-A- 19 955 375** | **US-A- 5 496 538** |

- **DATABASE PROMT [Online] STN INTERNATIONAL; "Airspray International Inc. (Packaging News)" Database accession no. 2002: 348596 XP002263847 & GLOBAL COSMETIC INDUSTRY; ISSN: 1523-9470, Bd. 170, Nr. 5, Mai 2002 (2002-05), Seite 59**
- **"F2 Finger Pump Foamer" Retrieved from the Internet: URL:www.airspray.net> [retrieved on 2006-07-06]**
- **"Degussa. Launches emulsion spray sunscreen" GLOBAL COSMETIC INDUSTRY, 1 May 2001 (2001-05-01), Retrieved from the Internet: URL: www/allbusiness.com/chemicals/specialt y-chemicals-industry/cosmetivs/80791...> [retrieved on 2009-03-20]**
- **H. Fiedler: "Der pharmazeutische Betrieb" 1996, vol. 9, , page 1525**
- **"sorbitan stearate" page 1-3, Retrieved from the Internet: URL:http:77chemicalland21.com/ lifescience/ foco/sorbitan%20stearate.htm> [retrieved on 2009-09-03]**
- **E. BARANY ET AL.: "Unexpected skin barrier influence from nonionic emulsifiers" INTERNATIONAL JOURNAL OF PHARMACEUTICS, vol. 195, 2000, pages 189-195,**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein System umfassend eine schäumbare lichtschutzwirksame kosmetische und dermatologische Zubereitung in einem mechanisch betriebenen Pump-Schaumspender, die eine erhöhte UV-A Schutzleistung aufweist. Die Zubereitung enthält amphiphil gecoatete organische UV-Lichtschutzfiltersubstanzen und Emulgatoren auf Basis von Alkylglycosiden.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut:
Die sogenannte UV-C-Strahlung mit einer Wellenlänge zwischen 100 und 280 nm wird von der Ozonschicht der Erdatmosphäre absorbiert und findet sich dementsprechend nicht im Sonnenspektrum. Sie ist daher ohne physiologische Bedeutung beim Sonnenbaden.

Der sogenannte UV-B-Bereich liegt zwischen 290 nm und 320 nm. UV-B-Strahlen sind wesentlich für die lang anhaltende Bräunung, der Haut verantwortlich, können aber gleichzeitig ein Erythem, einen einfachen Sonnenbrand oder sogar mehr oder weniger starke Verbrennungen verursachen. Auch chronische Lichtschäden, Photodermatosen und Herpes solaris können durch UV-B-Strahlung hervorgerufen werden.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluss der UV-B-Strahlung durch UV-A-Strahlung noch verstärkt werden.

So ist es u. a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Die neueren Erkenntnisse über die Wirkung der UV-A-Strahlen auf die Haut haben dazu geführt, daß man den Schutzmaßnahmen für diesen Strahlenbereich nun erhöhte Aufmerksamkeit widmet. Praktisch kein Sonnenschutzprodukt kommt mehr ohne wirksame UV-A-Filterwirkung aus, reine UV-B-Filterpräparate sind selten.

Beim. Auftragen eines Sonnenschutzmittels auf die Haut können die ultravioletten Strahlen durch zwei Effekte abgeschwächt werden: zum einen durch Reflexion und Streuung der Strahlen an der Oberfläche von pulverförmigen Feststoffen (physikalischer Lichtschutz) und zum anderen durch Absorption an chemischen Substanzen (chemischer Lichtschutz). Je nachdem, welcher Wellenlängenbereich absorbiert wird, unterscheidet man zwischen UV-B-Filtern (Absorptionsbereich 280 bis 320 nm), UV-A-Filtern (Absorptionsbereich 320 bis 400 nm) und Breitbandfiltern (Absorptionsbereich 290 bis ca. 380 nm).

Zum Schutz gegen UV-B-Strahlung sind zahlreiche Verbindungen bekannt, wobei deren Absorptionsmaximum möglichst um 308 nm liegen sollte, da hier die höchste Erythemwirksamkeit der Sonnenstrahlung vorliegt. Typische UV-B-Filter sind z. B. Derivate des 3-Benzylidencamphers, der 4-Aminobenzoesäure, der Zimtsäure, der Salicylsäure, des Benzophenons sowie auch des 2-Phenylbenzimidazols.

Auch zum Schutz gegen UV-A-Strahlung sind einige Verbindungen bekannt, wie insbesondere Dibenzoylmethanderivate. Allerdings sind Dibenzoylmethanderivate in der Regel nicht photostabil, weshalb kosmetische oder dermatologische Zubereitungen mit einem Gehalt an dieser Substanz zweckmäßigerweise auch bestimmte UV-Stabilisatoren enthalten sollten. Weitere bekannte UV-A-Filtersubstanzen sind bestimmte wasserlösliche, sulfonierte UV-Filtersubstanzen, wie z. B. Phenylen-1,4-bis-(2-benzimidazyl)-3,3'-5,5'tetrasulfonsäure und ihre Salze.

Neben den reinen UV-A- oder UV-B-Filtern gibt es Substanzen, die beide Bereiche abdecken. Zu dieser Gruppe der Breitbandfilter gehören beispielsweise unsymmetrisch substituierte s-Triazin-Verbindungen, wie z. B. das 2,4-Bis-{[4-(2-ethyl-hexyloxy)-2-hydroxy]-phenyl}-6-(4-methoxyphenyl)-1,3,5-triazin (INCI: Bis-Ethylhexyloxylphenol Methoxyphenyl Triazin), bestimmte Benzophenone, wie z. B. das 2-Hydroxy-4-methoxy-benzophenon (INCI: Benzophenone 3) oder das 2,2'-Methylen-bis-(6-(2H-benzotriazol-2-yl)-4-(1,1,3,3-tetramethylbutyl)-phenol) (INCI: Methylene Bis-Benzotriazolyl Tetramethylenebutylphenol).

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Da zur Charakterisierung einer Filtersubstanz nicht nur die Lage des Absorptionsmaximums, sondern vor allem der Absorptionsbereich wichtig ist, werden von jeder Substanz Absorptionsspektren aufgenommen. Für die Dosierung der Substanzen in den fertigen Formulierungen können die Extinktionswerte aber allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstariz in und auf der Hornschicht der Haut verteilt ist.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Die Einsatzkonzentration bekannter als Feststoff vorliegender Lichtschutzfiltersubstanzen, die insbesondere auch im UV-A-Bereich eine hohe Filterwirkung zeigen, ist allerdings häufig - gerade in Kombination mit anderen zu lösenden Substanzen - begrenzt. Es bereitet daher gewisse formulierungstechnische Schwierigkeiten, höhere Lichtschutzfaktoren bzw. UV-A-Schutzleistung zu erzielen.

Um einen optimalen UV-Schutz zu gewährleisten, müssen UV-Filtersubstanzen selbstverständlich in gelöster Form vorliegen. Bestimmte UV-Filtersubstanzen - wie Benzotriazole, Triazin- und Benzoxazol-Derivate - zeichnen sich an sich durch gute Lichtschutzwirkung aus. Ihr Hauptnachteil liegt allerdings darin, daß sie schlecht in üblichen Ölkomponenten löslich sind. Ein Nachteil des Standes der Technik ist dementsprechend, daß mit diesen Filtersubstanzen in der Regel nur vergleichsweise niedrige Lichtschutzfaktoren erreicht werden konnten, da ihre Löslichkeit oder Dispergierbarkeit in den Formulierungen zu gering ist, d. h. sie lassen sich nicht oder nur schwer in befriedigender Weise in solche Formulierungen einarbeiten.

Üblicherweise werden Lichtschutzformulierungen in Form von Cremes, Milchen, Gelen, Ölen und Lotionen dargereicht. In der Regel ist es schwer, schäumende Lichtschutzformulierungen, die gleichzeitig einen ausreichenden UV-Schutz aufweisen, herzustellen. Um die Formulierungen aufschäumen zu können, müssen Ihnen Emulgatoren zugesetzt werden. Zu den gut schäumenden Emulgatoren gehören die ionischen Emulgatoren wie z.B. Natriumlaurethsulfat. Ionische Emulgatoren sind jedoch nicht besonders hautverträglich, weshalb sie nur in geringen Konzentrationen eingesetzt werden können. Dies geht jedoch zu Lasten der Schaumqualität, Nichtionische Emulgatoren wie beispielsweise Fettalkoholethoxylate zeichnen sich zwar durch eine bessere Hautverträglichkeit aus, schäumen aber schlecht.

Schäume werden in der Regel in Sprühdosen (sog. Aerosoldosen) aus Aluminium oder Weißblech in Kombination mit einem Treibgas (Propan/Butan) angeboten. Diese Sprühdosen dürfen aus Sicherheitsgründen jedoch nur in einer Umgebungstemperatur von unter 50 °C aufbewahrt und eingesetzt werden. Diese Rahmenbedingung kann im alltäglichen Gebrauch von Sonnenschutzmitteln (z.B. am Strand) jedoch nicht immer eingehalten werden. Die Patentanmeldung DE 199 55 375 beschreibt eine Sonnenschutzzubereitung, die in einem Kolbentriebenen Schaumspender konditioniert sein können.

Es war daher die Aufgabe der vorliegenden Erfindung, hautfreundliche, gut aufschäumbare Sonnenschutzzubereitungen mit hohem Lichtschutzfaktor zu entwickeln, die auch im alltäglichen Gebrauch ohne Gefahr sehr einfach für den Anwender appliziert vund eingesetzt werden können.

Eine weitere Aufgabe der vorliegenden Erfindung war es eine lichtschutzwirksame kosmetische oder dermatologische Zubereitung zur Verfügung zu stellen, die eine optimale Verteilung von Lichtfiltersubstanzen auf der Haut ermöglicht und die bei der Verteilung auf der Haut als sensorisch angenehm empfunden wird.

Es war überraschend und für den Fachmann nicht vorauszusehen, dass schäumbare kosmetische und/oder dermatologische Sonnenschutzzubereitungen gemäß vorliegendem Anspruch 1, die

a) mindestens einen partikulären organischen Lichtschutzfilter,
b) mindestens eine weitere UV-A-, UV-B- und/oder Breitbandfiltersubstanz und
c) mindestens einen nichtionischen Emulgator

enthalten, neben anderen kosmetischen und/oder dermatologischen Hilfs- und Zusatzstoffen, die in einem mechanischen Schaumdispenser gemäß vorliegendem Anspruch 1 vorliegen, den Mängeln des Standes der Technik abhelfen.

Zwar beschreibt auch die EP 0 793 955 schäumbare Sonnenschutzzubereitungen, doch konnte diese Schrift nicht den Weg zur vorliegenden Erfindung weisen, da in ihr gerade die nicht unbedenklichen unter Druck stehenden Treibgasdosen zum Einsatz kommen. Eine andere Schrift (WO 01/35904) beschreibt zwar schäumbare Sonnenschutzzubereitungen, die auch mechanische Schaumdispenser umfasst, jedoch werden hier amphotere Emulgatoren verwendet, deren Schaumverhalten von minderer Qualität ist. Des weiteren konnte diese Schrift nicht den Weg zur Verarbeitung partikulärer Lichtschutzfilter wie z.B. das in Partikelform vorliegende 2,2'-Methylen-bis-(6-(2H-benzotriazol-2-yl)-4-(1,1,3,3-tetramethylbutyl)-phenol) aufzeigen. Das US-Patent US 6,143,282 beschreibt zwar schäumbare Lichtschutzformulierungen die auch Benztriazolderivate enthalten, doch auch hierbei handelt es sich um lösliche Lichtschutzfilter auf Siliziumbasis, die darüber hinaus nur in Kombination mit Zimtsäureund/oder Salicylsäurederivaten zu stabilen Sonnenschutzzubereitungen verarbeitet werden können.

Die in der vorliegenden Erfindung genutzten partikulären organischen Lichtschutzfilter begünstigen aber aufgrund ihrer grenzflächenaktiven Hülle gerade in überraschender Weise die Schaumbildung und bewirken aufgrund ihrer hohen UV-Filterleistung einen außergewöhnlich hohen UV-Lichtschutz, insbesondere einen außergewöhnlich hohen UV-A-Lichtschutz. Darüber hinaus bietet die erfindungsgemäße Zubereitung einen ungewöhnlich hohen Schutz vor vorzeitiger Hautalterung.

Die erfindungsgemäßen Sonnenschutzzubereitungen enthalten vorteilhafter Weise partikuläre organische Lichtschutzfilter in einer Menge von 0,01 bis 20 Gewichts-% und insbesondere in einer Menge von 0,5 bis 10 Gewichts-%, jeweils bezogen auf das Gesamtgewicht der Zubereitung.

Es ist erfindungsgemäß von Vorteil als partikuläre organische Lichtschutzfilter amphiphile Filter mit dem Grundgerüst des Benztriazols einzusetzen, wobei das besonders bevorzugte Benzotriazolderivat das 2,2'-Methylen-bis-(6-(2H-benzotriazol-2-yl)-4-(1,1.,3,3-tetramethylbutyl)-phenol) [z.B. Tinosorb M (Ciba)] ist.

Es ist außerdem erfindungsgemäß vorteilhaft, in den Sonnenschutzzubereitungen nichtionische Emulgatoren in einer Konzentration von 0,01 bis 20 Gewichts-% und insbesondere in einer Konzentration von 0,5 bis 10 Gewichts-%, jeweils bezogen auf das Gesamtgewicht der Zubereitung einzusetzen.

Erfindungsgemäß sind Alkylpolyglucoside die nichtionischen Emulgatoren, die beispielsweise in der DE 19723733.9 beschrieben sind. Erfindungsgemäß besonders bevorzugt ist der Einsatz einer wirksamen Menge einer oder mehrerer nichtionischer, grenzflächenaktiver Substanzen, gewählt aus der Gruppe der Alkylglucoside, welche sich durch die Strukturformel

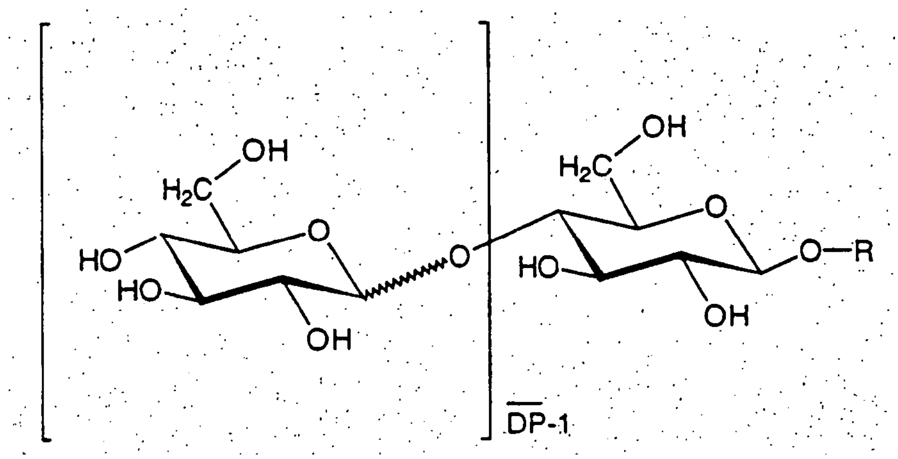

auszeichnen, wobei R einen verzweigten oder unverzweigten Alkylrest mit 4 bis 24 Kohlenstoffatomen darstellt, und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucosiden und ist definiert als

$$\overline{DP} = \frac{p_1}{100} \cdot 1 + \frac{p_2}{100} \cdot 2 + \frac{p_3}{100} \cdot 3 + \ldots = \sum_i \frac{p_i}{100} \cdot i$$

Dabei stellen $p_1$, $p_2$, $p_3$ ... bzw, $p_i$ den Anteil der einfach, zweifach dreifach ... i-fach glucosylierten Produkte in Gewichtsprozenten dar. Erfindungsgemäß vorteilhaft werden Produkte mit Glucosylierungsgraden von 1 - 2, insbesondere vorteilhaft von 1,1 bis 1,5, ganz besonders vorteilhaft von ungefähr 1,3 gewählt.

Der Wert DP trägt dem Umstande Rechnung, daß Alkylglucoside herstellungsbedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40 - 70 Gew.-%.

Vorteilhaft wird R gewählt aus der Gruppe der unverzweigten Alkylreste, wobei der Myristylrest, der Cetylrest, der Stearylrest und der Eicosylrest bevorzugt werden.

Erfindungsgemäß eingesetzte Alkylglucoside sind erhältlich durch Verfahren, wie sie beispielsweise in der DE-OS 40 40 655 und anderen Schriften beschrieben werden. Sie sind im Handel von verschiedenen Herstellern erhältlich.

Beispielsweise vorteilhaft ist es, Gemische aus Stearylglucosid und Cetylglucosid zu verwenden. Solche Mischungen sind im Handel beispielsweise unter der Warenbezeichnung Tego® Care CG 90 (Cetearylpolyglucosid) von der Gesellschaft Th.Goldschmidt KG erhältlich.

Durch den kombinierten Einsatz der Lichtfiltersubstanzen mit den Emulgatoren auf Basis von Alkylglycosiden ist eine Möglichkeit gegeben, die es gestattet stabile Schäume herzustellen.

Erfindungsgemäß werden diese schäumbaren Sonnenschutzzubereitungen mittels eines Schaumdispensers aufgeschäumt, der mit Hilfe eines kolbenbetriebenen Pumpsystems, die flüssige Sonnenschutzzubereitung mit hoher Geschwindigkeit durch ein siebartiges Gewebe führt und sie dadurch aufschäumt.

Erfindungsgemäß besonders bevorzugt ist dabei ein Schaumdispenser mit Spritzwasserschutz.

Bei dem Schaumdispenser handelt es sich vorzugsweise um kolbenbetriebene Pumpschäumer (z.B. die Pumpfoamer der Fa. Airspray). Das aufzuschäumende Medium befindet sich in einem flüsssigen Aggregatzustand in einem Behältnis, z.B. einer Flasche. vorzugsweise aus". Kunststoff, Glas oder Metall, oder einem flexiblen Beutel. Der eigentliche Pumpdispenser ist über ein Gewinde, eine Schnapp- oder Siegelverbindung fest mit dem Flüssigbehältnis verbünden. Zur Schaumerzeugung wird der Schaumkopf. manuell betätigt. Der manuelle Betätigungshub wirkt direkt oder indirekt auf eine Kolbenpumpe die das flüssige Medium über ein Steigrohr aus dem Flüssigkeitsbehältnis in einen Hubraum transportiert. Bei einem weiteren Betätigungshub wird das Füllgut aus dem Hubraum ausgetrieben und unter höher Luftanreicherung, die über eine zusätzliche Luftpumpe erzeugt und dem Füllgutstrom zugeführt wird, durch ein sieb- oder maschenartiges Gewebe getrieben, wodurch ein feinporiger fester Schaum entsteht, der über eine Dosieröffnung appliziert wird.

Die Zubereitungen im Sinne der vorliegenden. Erfindung enthalten mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure (2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Weitere vorteilhafte UV-A-Filtersubstanzen im Sinne der vorliegenden Erfindung sind Hydroxybenzophenone, welche sich durch die folgende Strukturformel, auszeichnen:

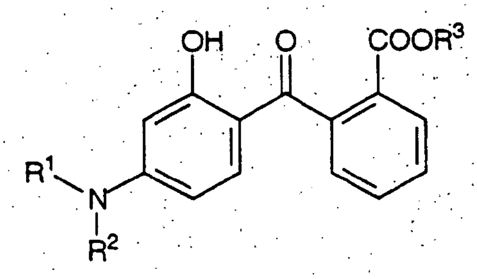

worin

- $R^1$ und $R^2$ unabhängig voneinander Wasserstoff, $C_1$-$C_{20}$-Alkyl, $C_3$-$C_{10}$-Cycloalkyl oder $C_3$-$C_{10}$-Cycloalkenyl bedeuten, wobei die Substituenten $R^1$ und $R^2$ gemeinsam mit dem Stickstoffatom, an das sie gebunden sind, einen 5- oder 6-Ring bilden können und
- $R^3$ einen $C_1$-$C_{20}$-Alkyl Rest bedeutet.

Ein besonders vorteilhaftes Hydroxybenzophenon im Sinne der vorliegenden Erfindung ist das 2-(4'-Diethylamino-2'-hydoxybenzoyl)-benzoesäurehexylester (auch: Aminobenzophenon), welches sich durch folgende Struktur auszeichnet

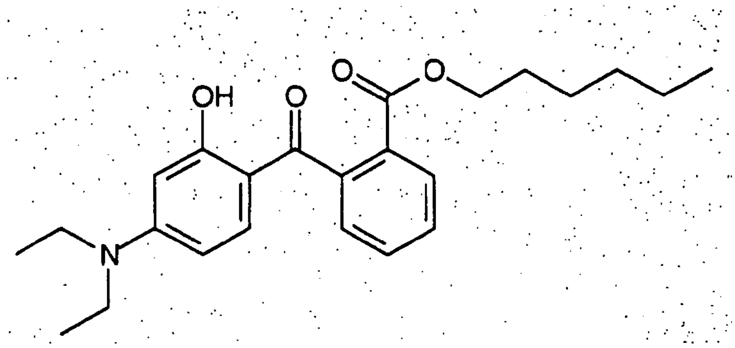

und unter dem Uvinul A Plus bei der Fa. BASF erhältlich ist.

Ebenso vorteilhafte UV-A Filtersubstanzen im Sinne der vorliegenden Erfindung, sind Benzoxazol-Derivate, die sich durch die folgende Strukturformel auszeichnen,

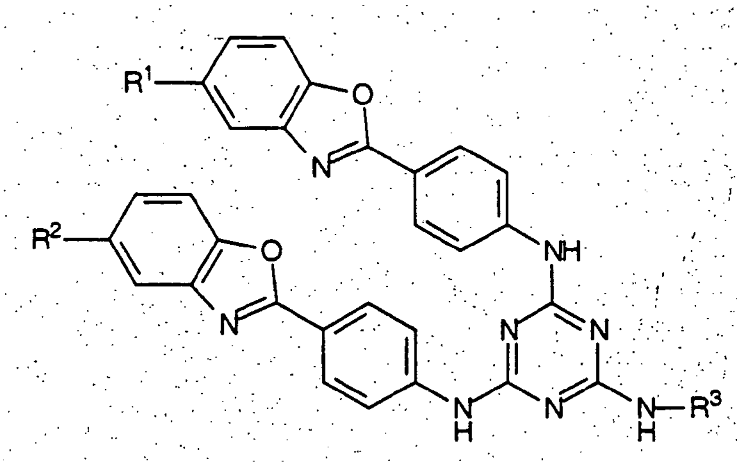

worin $R^1$, $R^2$ und $R^3$ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste $R^1$ und $R^2$ gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn $R^3$ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel

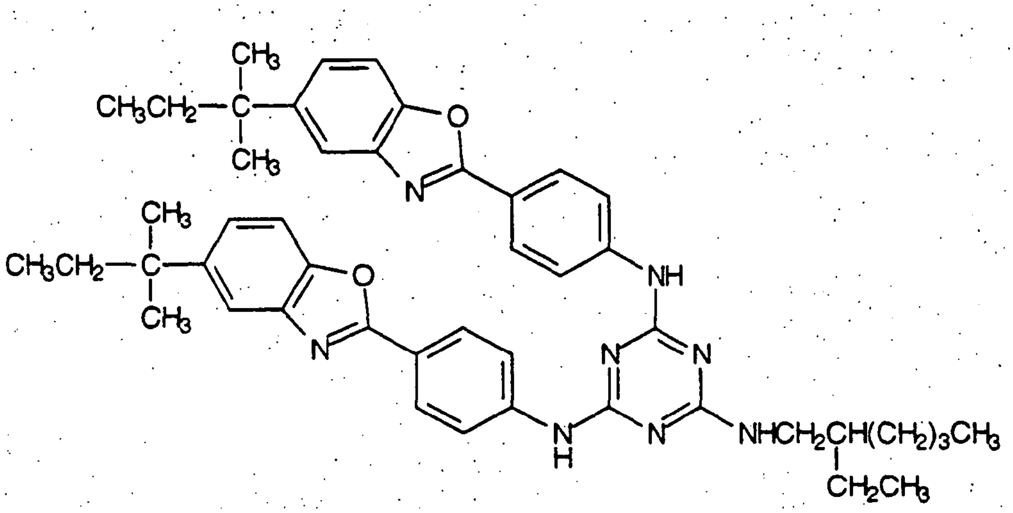

auszeichnet und bei 3V Sigma unter der Handelsbezeichnung Uvasorb® K2A erhältlich ist.

Weitere vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.
Vorteilhafte weitere UV-Filtersubstanzen im Sinne der vorliegenden Erfindung sind sulfonierte, wasserlösliche UV-Filter, wie z. B.:

- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenyldimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1 , 4-di(2-oxo-3-bornylidenmethyl-10-sulfonsäure) bezeichnet wird. Benzol-1,4-di(2-oxo-3-bornylidenmethyl-10-sulfonsäure) hat die INCl-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.

- 2,4-Bis-{(4-(2-Ethyl-hexyloxy)-2-hydroxy]-phenyl}-6-(4-methoxyphenyl)-1,3,5-triazin (INCl: Aniso Triazin), welches unter der Handelsbezeichnung Tinosorb® S bei der CIBA-Chemikalien GmbH erhältlich ist;
- Diethylhexylbutylamidotriazon (INCI: Diethylhexylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester); auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist. Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches unter der Handelsbezeichnung Mexoryl® XL bei der Fa. Chimex erhältlich ist.

Vorteilhafte öllösliche UV-B- und/oder Breitband-Filtersubstanzen im Sinne der vorliegenden Erfindung sind z. B.:

■ 3-Benzylidencampher-Derivate, vorzugsweise 3-(4-Methylbenzyliden)campher, 3-Benzylidencampher;

■ 4-Aminobenzoesäure-Derivate, vorzugsweise 4-(Dimethylamino)-benzoesäure(2-ethylhexyl)ester, 4-(Dimethylamino)benzoesäureamylester;

■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon

■ sowie an Polymere gebundene UV-Filter.

■ 3-(4-(2,2-bis Ethoxycarbonylvinyl)-phenoxy)propenyl)-methoxysiloxan/Dimethylsiloxan - Copolymer (Dimethycodiethyl benzalmalonat), welches beispielsweise unter der Handelsbezeichnung Parsol® SLX bei Hoffmann La Roche erhältlich ist.

Vorteilhafte wasserlösliche Filtersubstanzen sind z.B.:
Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren. Salze.

Eine weitere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Besonders vorteilhafte Zubereitungen im Sinne der vorliegenden Erfindung, die sich durch einen höhen bzw. sehr hohen UV-A- und/oder UV-B-Schutz auszeichnen, enthalten neben der oder den erfindungsgemäßen Filtersubstanz (en) bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethylhexyloxy)-2-hydroxy]-phenyl}-6-(4-methoxyphenyl)-1,3,5-triazin, jeweils einzeln oder in beliebigen Kombinationen miteinander.

Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$).

Die Pigmente können vorteilhaft im Sinne der vorliegenden Erfindung auch in Form kommerziell erhältlicher öliger oder wässriger Vordispersionen zur Anwendung kommen.

Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden.

Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen. Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO neutral | / | H&R |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul $TiO_2$) | Octyltrimethylsilan | Degussa |

Die Liste der genannten UV-Filter, die im Sinne der vorliegenden Erfindung eingesetzt werden können, soll

selbstverständlich nicht limitierend sein.

Vorteilhaft enthalten die erfindungsgemäßen Zubereitungen die Substanzen, die UV-Strahlung im UV-A- und/öder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Erfindungsgemäß können die kosmetischen und/oder dermatologischen Lichtschutzförmulierungen wie üblich zusammengesetzt sein und dem kosmetischen und/öder dermatologischen Lichtschutz, ferner zur Behandlung, der Pflege und der Reinigung der Haut und/oder der Haare dienen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Die kosmetischen und/oder dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Farbstoffe, Pigmente (z.B. $TiO_2$, ZnO, $BaSO_4$), die eine färbende Wirkung haben und/oder als anorganische . Lichtschutzfilter erfindungsgemäß vorteilhaft eingesetzt werden können. Weitere erfindungsgemäße Hilfsstoffe sind Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$ -Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, IDS (Iminodibernsteinsäure) und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoëharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 10 Gew.-%, besonders bevorzugt 0,05 - 7 Gew.-%, insbesondere 0,1 - 5 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001-5 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Darüber hinaus eignen sich ausgewählte erfihdungsgemäße Rezepturen, welche z. B. bekannte Antifaltenwirkstoffe wie Flavonglycoside (insbesondere $\alpha$-Glycosylrutin), Coenzym Q10, Vitamin E und/oder Derivate und dergleichen enthalten, insbesondere vorteilhaft zur Prophylaxe und Behandlung kosmetischer oder dermatologischer Hautveränderungen, wie sie z. B. bei der Hautalterung (z.B. Falten und Fältchen) auftreten. Weiterhin vorteilhaft eignen sie sich gegen das Erscheinungsbild der trockenen bzw. rauhen Haut.

Eine gegebenenfalls vorhandene Lipidphase kann vorteilhaft gewählt werden aus folgender Substanzgruppe:

- Mineralöle, Mineralwachse

- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxahe, Diphenylpolysiloxane sowie Mischformen daraus.

Eine Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen. Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, lsopropyloleat, n-Butylstearat, n-Hexyllaurat, n-Decyloleat, lsooctylstearat, lsononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, lsoeicosan, 2-Ethylhexylcocoat, $C_{12-15}$-Alkylbenzoat, Capryl-Caprinsäure-triglycerid, Dicaprylylether, Dicaprylyl Carbonat, Butylen Gylcol Dicaprylat/Dicaprat.

Besonders vorteilhaft sind Mischungen aus $C_{12-15}$-Alkylbenzoat und 2-Ethylhexylisostearat, Mischungen aus $C_{12-15}$-Alkylbenzoat und lsotridecylisononanoat sowie Mischungen aus $C_{12-15}$-Alkylbenzoat, 2-Ethylhexylisostearat und Isotridecylisononanoat.

Weitere vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ von CP Hall oder Corapan TQ von Haarmann & Reimer*).

Von den Kohlenwasserstoffen sind Paraffinöl, Squalan und Isoparaffin vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) oder Dimethicon als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyloder -monobutylether, Propylenglykolmonomethyl, -monoethyloder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdikkungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß ist auch ein mechanischer Schaumdispenser, enthaltend eine erfindungsgemäße schäumbare kosmetische und/oder dermatologische Sonnenschutzzubereitung.

Insbesondere ist es erfindungsgemäß, wenn ein solcher mechanischer Schaumdispenser mittels eines kolbenbetriebenen Pumpsystems die flüssige Sonnenschutzzubereitung mit hoher Geschwindigkeit durch ein siebartiges Gewebe führt und sie dadurch aufschäumt.

In einer erfindungsgemäß vorteilhaften Ausführungsform verfügt ein solch erfindungsgemäßer mechanischer Schaumdispenser über einen Spritzwasserschutz.

Erfindungsgemäß ist die Verwendung einer kosmetischen und/oder dermatologischen Sonnenschutzzubereitung nach einem der vorhergehenden Ansprüche zum Schutz vor UV-A-Strahlung und vorzeitiger lichtinduzierter Hautalterung.

Die nachfolgenden Beispiele sollen die vorliegende Erfindung verdeutlichen. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiele:**

**1. O/W Sonnenschutz Emulsionen**

| | 1 | 2 | 3 | 4 | 5 | 6 | 7 |
|---|---|---|---|---|---|---|---|
| Glycerinmonostearat SE | 0,50 | 1,00 | 3,00 | | | 1,50 | |
| Glyceryl Stearat Citrat | 2,00 | | | 1,00 | 2,00 | | 2,50 |
| Stearinsäure | | 3,00 | 0,75 | 2,00 | | | |
| PEG-40 Stearat | 0,50 | | | | | 2,00 | |
| PEG-100 Stearat | | | 1,50 | | | | |
| Cetyl Dimethicon Copolyol | | | | 0,75 | | 0,50 | |
| Cetyl Phosphat | | | 0,75 | | 1,00 | | |
| Cetearylpolyglucosid | 0,50 | 1,50 | 3,00 | 1,80 | 2,50 | 0,25 | 2,50 |
| Stearyl Alkohol | | | 3,00 | | | 2,00 | 0,50 |
| Cetyl Alkohol | 2,50 | 1,00 | | | 0,50 | | 2,00 |
| UVASorb® K2A | | 2,50 | 3,00 | 4,00 | | | |
| Aminobenzophenon (Uvinul®A+) | | | | 2,50 | | | |
| Butylmethoxydibenzoylmethan | | | 2,00 | | | | |
| Bis-Ethylhexyloxyphenol Methoxyphenyl Triazin | 3,00 | | 1,00 | 0,25 | 5,00 | | |
| Phenylbenzmidazol Sulfonsäure | | 0,50 | 1,50 | | | 2,00 | |
| Ethylhexyl Triazon | 2,00 | | | 2,00 | | 2,00 | |
| Diethylhexyl Butamido Triazon- | | 2,00 | | | | | |
| Ethylhexyl Methoxycinnamat | | 3,50 | | 10,00 | | | |
| Octocrylen | | | | 5,00 | 9,00 | 7,50 | 2,50 |
| Methylen Bis-Benztriazolyl Tetramethylbutylphenol | 2,00 | 1,70 | 0,50 | 4,50 | 2,50 | 1,50 | 3,00 |
| Ethylhexylsalicylat | | | 3,00 | | | | 5,00 |
| Drometrizol Trisiloxan | | | 0,5 | | | 1,00 | |
| Titandioxid T 805 | | 1,50 | | | 1,00 | 0,50 | |

(fortgesetzt)

| | 1 | 2 | 3 | 4 | 5 | 6 | 7 |
|---|---|---|---|---|---|---|---|
| Titandioxid MT-100Z | 1,00 | | | 3,00 | 1,00 | | |
| C12-15 Alkyl Benzoat | | 2,50 | | | | 7,00 | 5,00 |
| Dicaprylyl Ether | | | 3,50 | | 2,00 | | |
| Butylenglycol Dicaprylat/Dicaprat | 5,00 | | | 5,00 | 3,00 | | |
| Cetearyl Isononanoate | | 4,00 | | | | 2,00 | 2,00 |
| Diethylhexylnaphthalat | | | 5,50 | | | | |
| Dimethicon | | 0,50 | 1,00 | | 2,00 | | |
| Cyclomethicon | 2,00 | | | 4,50 | | | 0,50 |
| Shea Butter | | 2,00 | | | | | 0,50 |
| PVP Hexadecen Copolymer | 0,50 | | | 0,50 | 1,00 | | 1,00 |
| Glycerin. | 3,00 | 7,50 | | 7,50 | 5,00 | | 2,50 |
| Carbomer | | | | 0,50 | | | |
| Acrylates/C10-30Alkyl Acrylate Crosspolymer | | 1,00 | | | | 0,50 | |
| Xanthan Gummi | 0,15 | | 0,05 | | | | 0,30 |
| Butylene Glycol | | 5,00 | | | | 7,00 | |
| Vitamin E Acetat | 0,50 | | 0,25 | 0,50 | 0,75 | | 1,00 |
| Alpha-Glucosylrutin | 0,25 | | | 0,20 | | 0,25 | |
| Fucogel®1000 | | | 1,50 | | | 5,00 | |
| Dihydroxyaceton | | 1,50 | | | | | 3,00 |
| DMDM Hydantoin | | 0,60 | 0,40 | 0,20 | | | |
| Methylparaben | 0,15 | | 0,25 | | 0,50 | | |
| Phenoxyethanol | 1,00 | 0,40 | | 0,40 | 0,50 | | 0,60 |
| EDTA | | 0,20 | 0,35 | 0,50 | 0,02 | | 0,03 |
| Ethanol | | 2,00 | 1,50 | | 3,00 | 5,00 | 1,00 |
| Insekt Repellent 3535 | | | | | | 10,00 | |
| Parfüm | 0,20 | 0,20 | | | | 0,30 | 0,40 |
| Wasser | ad 100 | ad 100 | ad 100 | ad 100 | ad 100 | ad 100 | ad 100 |

**2. Hydrodispersionen**

| | 1 | 2 | 3 | 4 | 5 |
|---|---|---|---|---|---|
| PEG- 100 Stearat | 1,00 | | | 0,5 | |
| Cetyl Alkohol | | | | 1,00 | |
| Natrium Carbomer | | 0,20 | | 0,30 | |
| Acrylates/C10-30 Alkyl Acrylate Crosspolymer | | | 0,40 | 0,10 | 0,10 |
| Xanthan Gummi | 0,50 | 0,30 | 0,15 | | 0,50 |

(fortgesetzt)

| | 1 | 2 | 3 | 4 | 5 |
|---|---|---|---|---|---|
| Dimethicon / Vinyl Dimethicon Crosspolymer | | | 5,00 | | 3,00 |
| UVASorb® K2A | | 1,50 | | 3,50 | |
| Bis-Ethylhexyloxyphenol Methoxypheny Triazin | 1,00 | 1,50 | | | 1,00 |
| Terephthaliden Dicampher Sulfonsäure | | 0,20 | | | 0,50 |
| Phenylbenzmidazol Sulfonsäure | | | 1,00 | 2,00 | |
| Uvinul® A Plus | 3,00 | | | | 0,50 |
| Ethylhexyl Methoxycinnamat | | | | 5,00 | 8,00 |
| Butyl Methoxydibenzoylmethan | 1,00 | | | 0,50 | 0,50 |
| Methylen Bis-Benzotriazolyl Tetramethylbutylphenol | 3,00 | 5,00 | 0,50 | 2,50 | 1,00 |
| Ethylhexyl Triazon | 4,00 | | 3,00 | 4,00 | |
| Octocrylen | | 4,00 | 10,00 | | 2,50 |
| Drometrizol Trisiloxan | 3,00 | | | | |
| Titandioxid T805 ® | 0,50 | | 2,00 | 3,00 | 1,00 |
| Zinkoxid NDM ® | 2,00 | 4,00 | | | |
| C12-15 Alkyl Benzoate | 2,00 | 2,50 | | | |
| Cetearylpolyglucosid | 0,25 | 1,00 | 0,50 | 2,50, | 3,00 |
| Butylenglycol Dicaprylat/Dicaprat | 4,00 | | | 6,00 | |
| Dicaprylyl Carbonat | | 2,00 | | | |
| Dimethicon | | 0,50 | 12,50 | 4,50 | 7,00 |
| Cyclomethicon | 10,00 | 2,00 | | 2,50 | 10,00 |
| Shea Butter | | 2,00 | | | |
| PVP Hexadecen Copolymer | 0,50 | | | 0,50 | 1,00 |
| Ethylhexyloxyglycerin | | 0,50 | 1,00 | | 0,50 |
| Glycerin | 3,00 | 7,50 | | 7,50 | 2,50 |
| Glycin Soja | | 1,50 | 1,00 | | |
| Vitamin E Acetat | 0,50 | 0,20 | 0,25 | 0,75 | 1,00 |
| $\alpha$ - Glycosil Rutin | | 0,30 | | 0,25 | |
| Trinatrium EDTA | | 0,30 | 0,10 | 0,20 | |
| Konkaben LMB ® | 0,20 | | | | 0,15 |
| Methylparaben | 0,50 | | | 0,15 | |
| Phenoxyethanol | 0,50 | | | 1,00 | 0,60 |
| Ethanol | 3,00 | 7,00 | 3,50 | | 1,00 |
| Dihydroxyaceton | 3,50 | | | | |
| Parfüm | 0,20 | | 0,20 | 0,40 | 0,20 |
| Farbstoffe, wasserlöslich | | | 0,02 | | |
| Wasser | ad 100 | ad 100 | ad 100 | ad 100 | ad 100 |

**3. Sprühbare Emulsionen**

| | 1 | 2 | 3 | 4 | 5 | 6 | 7 | 8 |
|---|---|---|---|---|---|---|---|---|
| Glycerinmonostearat SE | 0,50 | 2,00 | 3,00 | 5,00 | | | 0,50 | 4,00 |
| Glyceryl Isostearat | | | | | 3,50 | 4,00 | 2,00 | |
| Isoceteth-20 | | 0,50 | | | 2,00 | | | |
| Ceteareth-12 | | 5,00 | | 1,00 | | | | 3,50 |
| Ceteareth-20 | | | | 2,00 | | 2,50 | 3,00 | |
| PEG-100 Stearat | 5,00 | | 1,00 | | | | | 0,50 |
| Cetyl Alkohol | 2,50 | 1,00 | | 1,50 | | 0,50 | 1,50 | |
| Cetyl Palmitat | | | | 0,50 | | 1,00 | | |
| Cetyl Dimethicon Copolyol | 0,50 | | | | 0,50 | | 1,00 | |
| Cetearylpolyglucosid | 0,50 | 1,50 | 2,00 | 3,00 | 2,50 | 4,00 | 1,75 | 1,00 |
| Polyglyceryl-2 Dipolyhydroxystearat | | | | 0,75 | 0,25 | | | |
| UVASorb® K2A | 1,50 | | 2,00 | 3,00 | 5,00 | | | |
| Uvinul® A+ | | | | | | | | |
| Bis-Ethylhexyloxyphenol Methoxyphenyl Triazin | 2,00 | 1,40 | | 0,75 | | | 2,00 | |
| Dinatrium Phenyl Dibenzimidazol Tetrasulfonat | | 2,00 | | | 1,00 | | | 2,00 |
| Terephthaliden Dicampher Sulfonsäure | | | 0,50 | | | | 1,00 | |
| Butyl Methoxydibenzoylmethan | 1,50 | | 1,00 | | | | 0,75 | |
| Drometrizol Trisiloxan | | | 2,00 | | | 3,00 | | 1,00 |
| Methylen Bis-Benzotriazolyl Tetramethylbutylphenol | 1,50 | 4,50 | 0,50 | 1,75 | 3,00 | 2.00 | 2.50 | 5.00 |
| Ethylhexyl Methoxycinnamat | 8,00 | | | 4,50 | 5,00 | 10,00 | | |
| Diethylhexyl Butamido Triazon. | | | | | 2,00 | 2,00 | | 1,50 |
| Ethylhexyl Triazon | | | 2,00 | 4,00 | | 2,00 | 1,50 | 3,00 |
| Octocrylen | | | 5,00 | | 10,00 | | | 7,50 |
| C12-15 Alkyl Benzoat | 3,50 | | | | | | | |
| Cocoglyceride | | 3,00 | | 3,00 | | | | .3,50 |
| Dicaprylyl Ether | 4,00 | | 2,00 | | | | | |
| Butylenglycol Dicaprylat/Dicaprat | | 4,00 | | | | 3,00 | | |
| Dicaprylyl Carbonat | | | 5,00 | | | | | 6,00 |
| Phenyltrimethicon | 2,00 | | | | 2,00 | | | |
| PVP Hexadecen Copolymer | | | | 1,00 | 1,50 | | | |
| Glycerin | 10,0 | 5,00 | | 7,50 | | 10,00 | | |
| Fucogel®1000 | | | 2,50 | 6,00 | | | | |
| Tocopherol | 1,00 | | | 0,75 | 0,50 | | 1,00 | |
| Sonnenblumenöl | | 2,00 | 3,50 | | | | | 0,50 |
| Dihydroxyaceton | | | 2,00 | | | 1,50 | | |

(fortgesetzt)

| | 1 | 2 | 3 | 4 | 5 | 6 | 7 | 8 |
|---|---|---|---|---|---|---|---|---|
| Iodopropyl-Butylcarbamat | 0,12 | | | | 0,20 | | | |
| DMDM Hydantoin | | | | 0,10 | | | | |
| Methylparaben | | 0,50 | 0,25 | | 0,45 | | | |
| Phenoxyethanol | 0,50 | 0,40 | | 1,00 | | | | 1,00 |
| Ethylhexyloxyglycerin | | 0,30 | | | 1,00 | 0,35 | | |
| Ethanol | | | | 2,00 | | 6,00 | 7,50 | 4,00 |
| Trisodium EDTA | | 0,40 | | 0,15 | | 0,20 | | 0,50 |
| Parfüm | 0,20 | | 0,20 | 0,20 | 0,45 | | | 0,20 |
| Wasser | ad. 100 | ad. 100 | ad. 100 | ad. 100 | ad. 100 | ad. 100 | ad. 100 | ad. 100 |

## Patentansprüche

**1.** Schäumbare kosmetische und/oder dermatologische Sonnenschutzzubereitung, enthaltend

a) mindestens einen partikulären organischen Lichtschutzfilter,
b) mindestens eine weitere UV-A-, UV-B- und/oder Breitbandfiltersubstanz und
c) mindestens einen nichtionischen Emulgator,
neben anderen kosmetischen und/oder dermatologischen Hilfs- und Zusatzstoffen,
die in einem mechanischen Schaumdispenser vorliegt, **dadurch gekennzeichnet, dass** der Schaumdispenser ein kolbenbetriebene Pumpschäumer ist sowie **dadurch gekennzeichnet dass** es sich bei dem Emulgator um ein Alkylpolyglucosid handelt.

**2.** Schäumbare kosmetische und/oder dermatologische Sonnenschutzzubereitung nach Anspruch 1 enthaltend

a) partikuläre organische Lichtschutzfilter in einer Menge von 0,01 bis 20 Gewichts-%, insbesondere in einer Konzentration von 0,5 bis 10 Gewichts-%
b) nichtionische Emulgatoren in einer Konzentration von 0,01 bis 20 Gewichts-% und insbesondere in einer Konzentration von 0,5 bis 10 Gewichts-%
jeweils bezogen auf das Gesamtgewicht der Zubereitung.

**3.** Kosmetische und/oder dermatologische Sonnenschutzzubereitung nach einem der Ansprüche 1 oder 2, **dadurch gekennzeichnet dass** es sich bei dem partikulären organischen Lichtschutzfilter um amphiphile Filter mit dem Grundgerüst des Benztriazols handelt.

**4.** Kosmetische und/oder dermatologische Sonnenschutzzubereitung nach einem der Ansprüche 1 bis 3, **dadurch gekennzeichnet dass** es sich bei dem partikulären Lichtschutzflter um 2,2'-Methylen-bis-(6-(2H-benzotriazol-2-yl)-4-(1,1,3,3-tetramethylbutyl)-phenol) handelt.

**5.** Kosmetische und/oder dermatologische Sonnenschutzzubereitung nach einem der Ansprüche 1 bis 5, **dadurch gekennzeichnet dass** es sich bei dem Emulgator um Cetearylpolyglycosid handelt.

**6.** Kosmetische und/oder dermatologische Sonnenschutzzubereitung nach einem der Ansprüche 1 bis 6, **dadurch gekennzeichnet, dass** der mechanische Schaumdispenser mittels eines kolbenbetriebenen Pumpsystems die flüssige Sonnenschutzzubereitung mit hoher Geschwindigkeit durch ein sieb- oder maschenartiges Gewebe führt und sie dadurch aufschäumt.

**7.** Kosmetische und/oder dermatologische Sonnenschutzzubereitung nach einem der Ansprüche 1 bis 7, **dadurch gekennzeichnet, dass** der Schaumdispenser über einen Spritzwasserschutz verfügt.

8. Kosmetische und/oder dermatologische Sonnenschutzzubereitung nach einem der Ansprüche 1 bis 8, **dadurch gekennzeichnet dass** der eigentliche Pumpdispenser des Schaumdispensers ist über ein Gewinde, eine Schnapp- oder Siegelverbindung fest mit dem Flüssigbehältnis, welches die Sonnenschutzzubereitung enthält, verbunden ist.

9. Kosmetische und/oder dermatologische Sonnenschutzzubereitung nach einem der Ansprüche 1 bis 9, **dadurch gekennzeichnet, dass** als Füllgutbehältnis eine Flasche aus Kunststoff, Glas oder Metall, oder ein flexibler Beutel eingesetzt wird.

10. Verwendung einer kosmetischen und/oder dermatologischen Sonnenschutzzubereitung nach einem der vorhergehenden Ansprüche zum Schutz vor UV-A-Strahlung und vorzeitiger lichtinduzierter Hautalterung.

## Claims

1. Foamable cosmetic and/or dermatological sunscreen composition, comprising

   a) at least one particulate organic photoprotective filter,
   b) at least one further UV-A, UV-B and/or broadband filter substance and
   c) at least one nonionic emulsifier,

   besides other cosmetic and/or dermatological auxiliaries and additives which are present in a mechanical foam dispenser, **characterized in that** the foam dispenser is a piston-driven pump foamer and also **characterized in that** the emulsifier is an alkyl polyglucoside.

2. Foamable cosmetic and/or dermatological sunscreen composition according to Claim 1, comprising

   a) particulate organic photoprotective filters in an amount of from 0.01 to 20% by weight, in particular in a concentration of from 0.5 to 10% by weight,
   b) nonionic emulsifiers in a concentration of from 0.01 to 20% by weight and in particular in a concentration of from 0.5 to 10% by weight,

   in each case based on the total weight of the composition.

3. Cosmetic and/or dermatological sunscreen composition according to one of Claims 1 or 2, **characterized in that** the particulate organic photoprotective filter is amphiphilic filters with the basic backbone of benzotriazole.

4. Cosmetic and/or dermatological sunscreen composition according to one of Claims 1 to 3, **characterized in that** the particulate photoprotective filter is 2,2'-methylenebis(6-(2H-benzotriazol-2-yl)-4-(1,1,3,3-tetramethylbutyl)-phenol).

5. Cosmetic and/or dermatological sunscreen composition according to one of Claims 1 to 4, **characterized in that** the emulsifier is cetearyl polyglycoside.

6. Cosmetic and/or dermatological sunscreen composition according to one of Claims 1 to 5, **characterized in that** the mechanical foam dispenser leads, by means of a piston-driven pump system, the liquid sunscreen composition at high speed through a sieve- or mesh-like fabric and thereby foams it.

7. Cosmetic and/or dermatological sunscreen composition according to one of Claims 1 to 6, **characterized in that** the foam dispenser has splashproof protection.

8. Cosmetic and/or dermatological sunscreen composition according to one of Claims 1 to 7, **characterized in that** the actual pump dispenser of the foam dispenser is firmly attached to the liquid container which contains the sunscreen composition via a screw thread, a snap connection or seal connection.

9. Cosmetic and/or dermatological sunscreen composition according to one of Claims 1 to 8, **characterized in that** a bottle made of plastic, glass or metal, or a flexible bag is used as fill-material container.

10. Use of a cosmetic and/or dermatological sunscreen composition according to one of the preceding claims for protection against UV-A radiation and premature light-induced skin ageing.

**Revendications**

**1.** Préparation antisolaire cosmétique et/ou dermatologique transformable en mousse, contenant

a) au moins un filtre photoprotecteur organique particulaire,
b) au moins une autre substance filtrant les UV-A, les UV-B et/ou à large bande et
c) au moins un émulsifiant non ionique,

en plus d'autres adjuvants et additifs cosmétiques et/ou dermatologiques, qui se trouvent dans un distributeur mécanique de mousse, **caractérisée en ce que** le distributeur de mousse est un applicateur de mousse actionné par piston ainsi que **caractérisée en ce que** l'émulsifiant consiste en un alkylpolyglucoside.

**2.** Préparation antisolaire cosmétique et/ou dermatologique transformable en mousse selon la revendication 1, contenant

a) des filtres photoprotecteurs organiques particulaires en une quantité de 0,01 à 20 % en poids, en particulier à une concentration de 0,5 à 10 % en poids
b) des émulsifiants non ioniques à une concentration de 0,01 à 20 % en poids et en particulier à une concentration de 0,5 à 10 % en poids

chaque fois par rapport au poids total de la préparation.

**3.** Préparation antisolaire cosmétique et/ou dermatologique selon la revendication 1 ou 2, **caractérisée en ce que** le filtre photoprotecteur organique particulaire consiste en des filtres amphiphiles présentant le squelette de base du benzotriazole.

**4.** Préparation antisolaire cosmétique et/ou dermatologique selon l'une quelconque des revendications 1 à 3, **caractérisée en ce que** le filtre photoprotecteur particulaire consiste en 2,2'-méthylène-bis(6-(2H-benzotriazol-2-yl)-4-(1,1,3,3-tétraméthylbutyl)-phénol).

**5.** Préparation antisolaire cosmétique et/ou dermatologique selon l'une quelconque des revendications 1 à 4, **caractérisée en ce que** l'émulsifiant consiste en cétéarylpolyglucoside.

**6.** Préparation antisolaire cosmétique et/ou dermatologique selon l'une quelconque des revendications 1 à 5, **caractérisée en ce que** le distributeur mécanique de mousse envoie au moyen d'un système de pompe actionné par piston la préparation antisolaire liquide à une grande vitesse à travers un tissu à maille ou de type tamis et la fait ainsi mousser.

**7.** Préparation antisolaire cosmétique et/ou dermatologique selon l'une quelconque des revendications 1 à 6, **caractérisée en ce que** le distributeur de mousse est doté d'une protection contre les projections d'eau.

**8.** Préparation antisolaire cosmétique et/ou dermatologique selon l'une quelconque des revendications 1 à 8, **caractérisée en ce que** le distributeur à pompe proprement dit du distributeur de mousse est assemblé solidement par un filetage, un assemblage par encliquetage ou scellement avec le récipient à liquide qui contient la préparation antisolaire.

**9.** Préparation antisolaire cosmétique et/ou dermatologique selon l'une quelconque des revendications 1 à 8, **caractérisée en ce qu'**on utilise comme récipient à liquide un flacon en matière plastique, verre ou métal, ou un sachet souple.

**10.** Utilisation d'une préparation antisolaire cosmétique et/ou dermatologique selon l'une quelconque des revendications précédentes, pour la protection contre le rayonnement UV-A et le vieillissement prématuré photo-induit de la peau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19955375
- EP 0793955 A
- WO 0135904 A
- US 6143282 A
- DE 19723733
- DE 4040655 A